# EUROPEAN PATENT SPECIFICATION

(11) **EP 4 013 477 B1**
(45) Date of publication and mention of the grant of the patent: **03.06.2026**
(21) Application number: 20751176.7
(22) Date of filing: 11.08.2020
(51) Int. Cl.: A61M 5/50, A61M 39/10, A61M 39/16

(54) **NON-REMOVABLE CONNECTION SYSTEM**
NICHT ABNEHMBARES VERBINDUNGSSYSTEM
SYSTÈME DE CONNEXION NON AMOVIBLE

(30) Priority: 13.08.2019 EP 19191551
(43) Date of publication of application: 22.06.2022
(73) Proprietor: Gambro Lundia AB, 226 43 Lund (SE)
(72) Inventor: VERNIN, Guillaume, 69330 MEYZIEU (FR)
(74) Representative: PGA S.p.A., Milano, Succursale di Lugano
(86) International application number: PCT/EP2020/072498
(87) International publication number: WO 2021/028434

(56) References cited:
- EP-A1- 2 620 178
- WO-A1-2016/012834
- WO-A1-2021/044403
- GB-A- 2 451 891
- US-A1- 2014 243 797
- US-A1- 2017 120 032

## Description

### FIELD OF THE INVENTION

The present invention relates to a non removable connection system for medical devices particularly configured to be connected to a fluid line, such as a blood line, an infusion line or a monitoring line. In particular said connection system may be connected to the fluid lines of a dialysis machine or may be directly attached to the dialysis machine or to an accessory of the disposable set/dialysis machine. The present invention also refers to a disposable comprising fluid lines of a blood treatment apparatus, such as a dialysis machine, and said connection system.

### BACKGROUND

Common connection systems for medical purposes are already known in the state of the art, for example used to connect fluid lines each other or to a medical machine. In blood treatment apparatuses, such as dialysis machines, connectors are commonly used for connecting fluid lines, for example for connecting in a fluid-tight manner return lines and access lines to respective vascular access systems of the patient, and/or for connecting infusion lines or monitoring lines to the blood or fluid circuit. In blood treatment apparatuses, Luer-type connectors are frequently used. Besides the fact of ensuring the fluid-tight sealing and the suitability to be used in the medical field, connectors are required, in specific cases, to avoid voluntary or involuntary disconnections: for example, where the connector is used on a blood line of a dialysis machine, a disconnection during an operative condition of the machine would lead to dangerous consequences potentially lethal for the patient. Any undesired disconnection may lead to sterility issues and requires the operator intervention, sometimes including the interruption of the treatment.

Document US8882726 is directed to a drug delivery device defining a reservoir for containing a liquid, such as a medicine. The device comprises a distal end presenting an end-piece traversed by a channel along a longitudinal axis. The end-piece is surrounded by a collar extending from the distal end, wherein the collar is provided with a plurality of outer longitudinal ridges running on the whole circumference of the collar: all the longitudinal ridges have each a sloped surface. The device also comprises a tubular wall having an inner duct, wherein an interface of the tubular wall is associable with the end-piece to define a fluid tight connection. The tubular wall is also provided with an outer flange configured to be engaged with a thread arranged in an inner lateral wall of the collar to allow axial engagement between the tubular wall and the end-piece of the device. The tubular wall also comprises a skirt provided with inner longitudinal projections able to cooperate with the longitudinal ridges and their sloped surfaces, to allow a screwing rotation of the tubular wall and to prevent unscrewing rotation of the same. The applicant points out some drawbacks affecting the device described in US8882726, from manufacturing to ease of use, as well as the impossibility of using it in combination with blood treatment apparatuses. In particular, such connector system would cause twist of the fluid line during connection, determining unwanted restrictions of the fluid line cross section, which might reduce drug or blood flow rate or determine damage of the fluid line itself. Moreover, the architecture of the connector comprises several undercuts and recesses, which makes production very complex and, therefore, expensive.

Document GB2451891A1 discloses a first connector releasably secured to a second connector formed by a collar rotatably mounted over and about an insert. Male and female key elements provide means to avoid disangagement of connectors. Document US20140243797A1 discloses a male Luer connector including a body having a first end configured to be connected to a fluid line, a second end opposed to the first end, and a body longitudinal axis. The second end is configured to be inserted into and form a Luer slip connection with a female Luer connector. The male Luer connector also includes a locking collar that is connected to the body in a manner such that the locking collar rotates about the longitudinal axis. Document EP2620178A1 discloses a connector for medical infusion lines, transfusion lines, and the like, comprising a tubular body having at one end a male connector including an inner tubular element and an outer hollow element, which is internally threaded and can be coupled by screwing to a complementary female connector. The line connector further includes an outer manoeuvring sleeve coupled in unidirectional rotation to the body in the direction corresponding to screwing of the male connector with respect to the complementary female connector and free to turn in the opposite direction. Document WO2016012834A1 discloses a cap for medical fluid lines and the like, characterized in that it comprises a hollow body within which a male or female connector accessible at one end of the body is coaxially housed, and is configured so as to inviolably obstruct the passage through said body. A unidirectional coupling is provided to lock in rotation the connector with respect to the body in the direction corresponding to the screwing of the connector and a complementary connector to be coupled therewith, and to enable free rotation of the connector in the opposite direction. Document US 2017/120032 A1 discloses a connecting structure for medical use includes a first connecting portion and a second connecting portion. The first connecting portion includes a threadedly engageable portion, a support portion and a sealing portion. The second connecting portion includes a threadedly engageable portion and a sealing portion. An operating cylinder is mounted on an outer periphery of the support portion. A torque limiting mechanism is disposed between the operating cylinder and the support portion.

### OBJECT OF THE INVENTION

The aim of this invention is therefore to at least partially solve one or more of the drawbacks and/or limitations of the previous solutions.

A first object is to provide a non removable connector for medical applications suitable for connecting disposable components, such as fluid lines. An aim is to avoid the need of multiple interventions and checks during the set-up of a medical apparatus and during the following treatment of a patient. A further aim is to reduce the risk for the connection to untighten, either due to human error or to other causes, such as mechanical/thermal stress on the connectors. Another object is to provide a non removable connector easy to produce and cheap. These objects and more, which will appear more from the following description, are substantially achieved by a connection system and a disposable set in accordance with one or more of the following claims.

An aspect refers to a non-removable connection system (100) for a medical apparatus, said connection system (100) comprising:
- a first connector (1) comprising a fluid coupling portion (2), a main abutment portion (5) arranged on an external lateral wall of the first connector (1) and defining a protrusion radially extending outward from the first connector (1), the first connector (1) being a Luer type male connector;
- a second connector (11) comprising a respective fluid coupling portion (12) connectable, in a fluid tight manner, to the fluid coupling portion (2) of the first connector (1), so that a fluid is able to internally flow between the first and the second connector (1, 11), the second connector (11) further comprising at least one locking member (14), the second connector (11) being a Luer type female connector;
- a collar (20) coupled to the first connector (1) and comprising: a respective abutment portion (25) configured to contact the main abutment portion (5) of the first male connector (1) when disposed in an abutment configuration, so that the collar (20) is axially constrained with respect to the first connector (1) at least along one axial direction; a respective at least one locking member (24) engageable to the at least one locking member (14) of the second connector (11) through an axial engagement displacement along an engagement direction (ED), in a coupled configuration the at least one locking member (24) of the collar (20) and the at least one locking member (14) of the second connector (11) preventing an axial disengagement displacement of the collar (20) with respect to the second connector (11), said axial disengagement displacement being a displacement in an opposite direction with respect to said axial engagement displacement, the collar (20) comprising a main body having tubular shape extending between a first opening (21) configured to receive the second connector (11), and a second opening (22) receiving by insertion the first connector (1), the collar (20) comprising a plurality of fingers (26) arranged at the first opening (21) angularly distributed about the axis of the collar (20), said fingers (26) carrying the at least one locking member (24) of the collar (20); and wherein the connection system (100) is configurable, through the axial engagement displacement, from:
- an unlocked condition, wherein the at least one locking member (24) of the collar (20) and the at least one locking member (14) of the second connector (11) are in an uncoupled configuration; to
- a locked condition wherein the at least one locking member (24) of the collar (20) and the at least one locking member (14) of the second connector (11) are in the coupled configuration, in said locked condition the collar (20) being irremovably engaged to the second connector (11) through said axial engagement displacement, and the at least one locking member (14) of the second connector (11) cooperating with the at least one locking member (24) preventing disengagement of the first connector (1) from the second connector (11), and wherein:
- the at least one locking member (14) of the second connector comprises at least one recess, and the at least one locking member (24) of the collar (20) comprises at least one protrusion, the at least one protrusion of the collar (20) being configured in the locked condition to engage the recess of the second connector (11) to define a non removable axial engagement, or
- the at least one locking member (14) of the second connector (11) comprises at least one protrusion, and the at least one locking member (24) of the collar (20) comprises at least one recess, the at least one protrusion of the second connector (11) being configured in the locked condition to engage the recess of the collar (20) to define a non removable axial engagement.

### DRAWINGS

Some embodiments and some aspects of the invention will be described below with reference to the attached drawings, provided for illustrative purposes only, wherein:
- Figures 1 and 2 are a perspective views of the connection system, disposed in a locked condition, according to the present invention;
- Figures 2A and 2B are a cross section views of the connection system of figure 2;
- Figures 3 is an exploded view of the connection system according to the present invention;
- Figure 3A is a cross section view of figure 3;
- Figure 4 is a perspective view of the connection system, disposed in an unlocked condition, according to the present invention;
- Figure 4A is a cross section view of figure 4;
- Figures 5 and 6 are different embodiments of a disposable set according to the present invention;
- Figure 7 is a perspective view of a further embodiment of the connection system;
- Figures 7A and 7B are cross section views of the embodiment of figure 7 in an unlocked and in a locked condition respectively;
- Figure 8 is a perspective view of an additional embodiment of the connection system;
- Figures 8A and 8B are cross section views of the embodiment of figure 8 in an unlocked and in a locked condition respectively;
- Figure 9 is a perspective view of another embodiment of the connection system;
- Figure 9A is a cross section view of the embodiment of figure 9 in an unlocked condition.

### DEFINITIONS

In this detailed description, corresponding parts illustrated in the various figures are indicated with the same numerical references. The figures may illustrate the invention by means of non-scale representations; therefore, parts and components illustrated in the figures relating to the object of the invention may relate exclusively to schematic representations.

### Upstream and/ downstream

The terms upstream and downstream refer to a direction or trajectory of advancement of a fluid configured to flow within the connector or along the fluid line during normal usage of the apparatus.

### Non-Removable

By the wording 'non-removable' is meant that the connection system is in a locked condition under normal use. As below explained, in the locked condition of the connection system at least one locking projection 24 of the collar 20 and at least one locking projection 14 of the second connector 11 are in a coupled configuration, and a hooking portion 23 of the collar 20 and a hooking portion 13 of the second connector 11 are in an engaged configuration; in said locked condition the collar 20 is irremovably engaged to the second connector 11 through an engagement rotation and the locking projection 14 and the hooking portion 13 of the second connector 11 respectively cooperating with the locking projection 24 and the hooking portion 23 of the collar 20 prevent disengagement of the first connector 1 from the second connector 11.

### DETAILED DESCRIPTION

### Connection system 100

Reference number 100 is directed to a connection system, as shown in figures from 1 to 4, for a medical apparatus: the connection system 100 may be used to connect, in a fluid tight manner, fluid lines 51 of a medical machine 200, such as a blood treatment apparatus, in particular a dialysis machine, wherein the fluid lines 51 may be a blood circuit 50a of a disposable set 50, an infusion line 54, a monitoring line 55, an access line 52 or a return line 53.

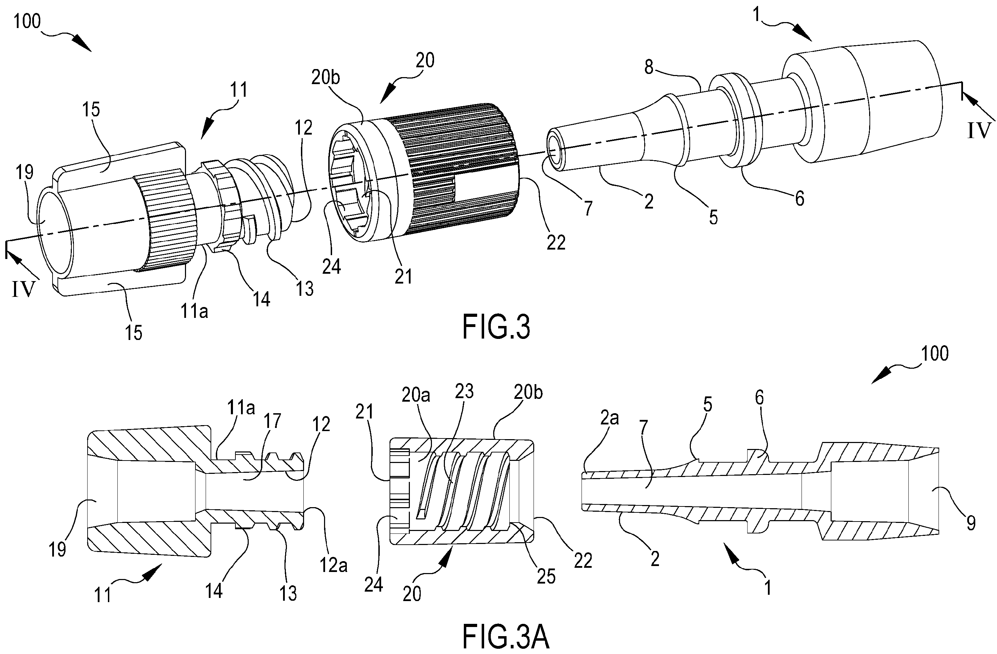

The connection system 100 comprises a first connector 1, shown in the exploded view of figure 3 and in the respective cross section in figure 3A, comprising a main body having tubular shape and defining an internal channel 7 for fluid passage: the main body of the first connector 1 and the internal channel 7 preferably extend in length along the same fluid flow axis, between a connection port 9 configured to be fixed/connected in a fluid tight manner to a fluid line 51 or directly to the medical machine 200 (or an accessory), and a coupling portion 2. The connection port 9 may be a male or female connection port. Usually, a tube end portion is irremovably fixed inside the connection port via e.g., gluing or bonding. The coupling portion 2 of the first connector 1 has preferably tubular shape and includes a tip portion 2a surrounding the internal channel: the coupling portion 2 has a tapered external surface, so that a diameter of the coupling portion 2 reduces towards the tip portion 2a of the coupling portion 2. In particular, the tapered external surface of the tip portion 2a of the coupling portion 2 has a taper angle comprised between 1° and 6°, more in particular comprised between 3° and 4°, more in detail the taper angle is equal to 3.44°. In an embodiment, the first connector 1, and in particular the coupling portion 2 of the first connector 1, is a Luer type connector: this means that dimensions of the coupling portion 2, the diameter of the internal channel 7 and the overall length of the first connector 1 are consistent with the Luer standards. In a non-limiting embodiment, the first connector 1 has a length comprised between 2 cm and 4 cm, and the internal channel 7 has an average diameter comprises between 2 mm and 3 mm.

The first connector 1 further comprises a main abutment portion 5 arranged on an external lateral wall of the main body, wherein the main abutment portion 5 defines a protrusion radially extending outward from the main body. The abutment portion 5 may have circular shape, for example a ring coupled to the main body extending outwardly. In an embodiment, the first connector 1 may further comprise an auxiliary abutment portion 6 arranged on the external lateral wall of the main body, wherein the auxiliary abutment portion 6 is axially distal from the main abutment portion 5 by an axial gap, so that a recess 8 is defined between the main abutment portion 5 and the auxiliary abutment portion 6. As shown in figures 3 and 3A, the auxiliary abutment portion 6 is axially interposed between the connection port 9 and the main abutment portion 5: similarly, the main abutment portion 5 is interposed between the coupling portion 2 and the auxiliary abutment portion 6.

In the shown embodiment, the first connector 1 is a male connector and it is made in one single piece preferably in plastic material, such as PVC, MBS, PC, or in metallic material, such as stainless steel. The connection system 100 further comprises a second connector 11, shown in the exploded view of figure 3 or in the cross section view of figure 3A, presenting a main body having tubular shape and defining an internal channel 17 for fluid passage: the main body of the second connector 11 and the internal channel 17 preferably extend in length, along the same fluid flow axis, between a connection port 19 configured to be fixed/connected in a fluid tight manner to a fluid line 51 or directly to the medical machine 200 (or an accessory), and a coupling portion 12. The connection port 19 may be a male or female connection port. Usually, a tube end portion is irremovably fixed inside the connection port 19 via e.g., gluing or bonding. The coupling portion 12 of the second connector 11 is configured to be connected to the coupling portion 2 of the first connector 1 in a fluid tight manner, so that a fluid is able to flow between the first and the second connectors 1, 11, and vice versa, in particular wherein the fluid is able to flow between the connection port 9 of the first connector 1 and the connection port 19 of the second connector 11, and vice versa. When the coupling portion 2 of the first connector 1 is connected to the coupling portion 12 of the second connector 11, the fluid flow axis of the first connector 1 coincides with the fluid flow axis of the second connector 11, and the internal channels 7, 17 respectively of the first and the second connectors 1, 11 define a unique internal channel for fluid passage extending from the connection port 9 of the first connector 1 to the connection port 19 of the second connector 11. The coupling portion 12 of the second connector **11** has preferably tubular shape and includes a terminal portion 12a surrounding the internal channel 17 and having a tapered internal surface, so that a diameter of the tapered internal surface increases towards the terminal portion 12a. According to the disclosed embodiment, the tapered internal surface of the terminal portion 12a of the second connector 11 is counter shaped to the tapered external surface of the tip portion 2a of the first connector 1. In other words, both the coupling portion 2 of the first connector 1 and the coupling portion 12 of the second connector 11 have conical shape. The tip portion 2a of the first connector 1 is configured to enter into and to contact the terminal portion 12a of the second connector 11, to define a fluid tight connection: the tapered external surface is configured to couple with the tapered internal surface to connect the first and the second connectors 1, 11 in a fluid tight manner. In particular, the tapered internal surface of the terminal portion 12a of the coupling portion 2 has a taper angle comprised between 1° and 6°, more in particular comprised between 3° and 4°, more in detail the taper angle is equal to 3.44°. In the specific embodiment, the taper angle of the coupling portion 2 of the first connector 1 is equal to the taper angle of the coupling portion 12 of the second connector 11. In an embodiment, the second connector 11, and in particular the coupling portion 12 of the second connector 11, is a Luer type connector: this means that dimensions of the coupling portion 12, the diameter of the internal channel 17 and the overall length of the second connector 11 are consistent with the Luer standards. In an embodiment, the second connector 11 has a length comprised between 2 cm and 3 cm, and the internal channel 17 has an average diameter comprises between 3 mm and 4 mm.

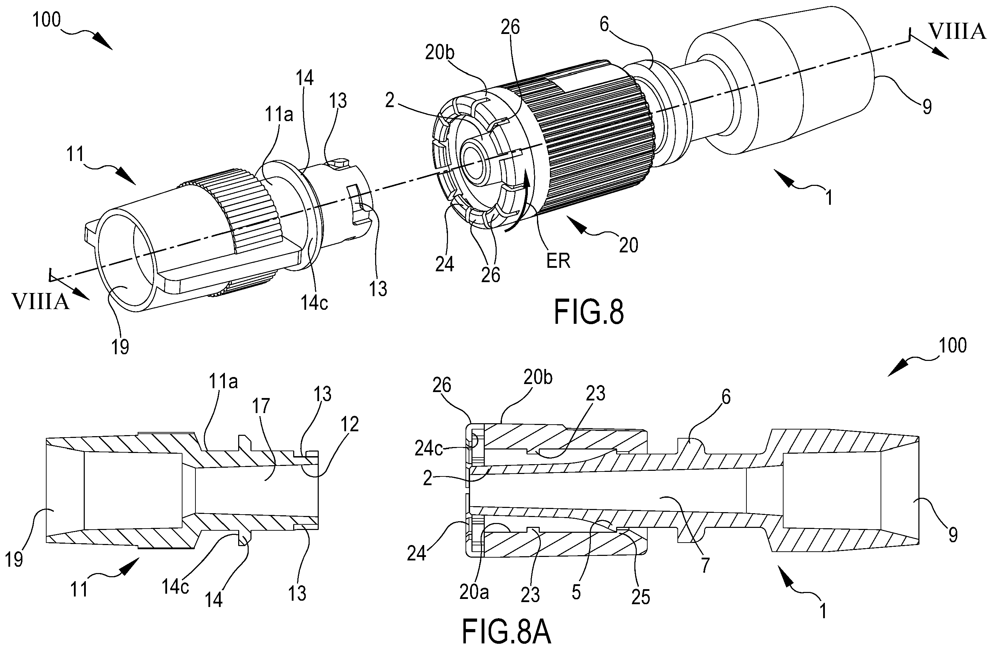

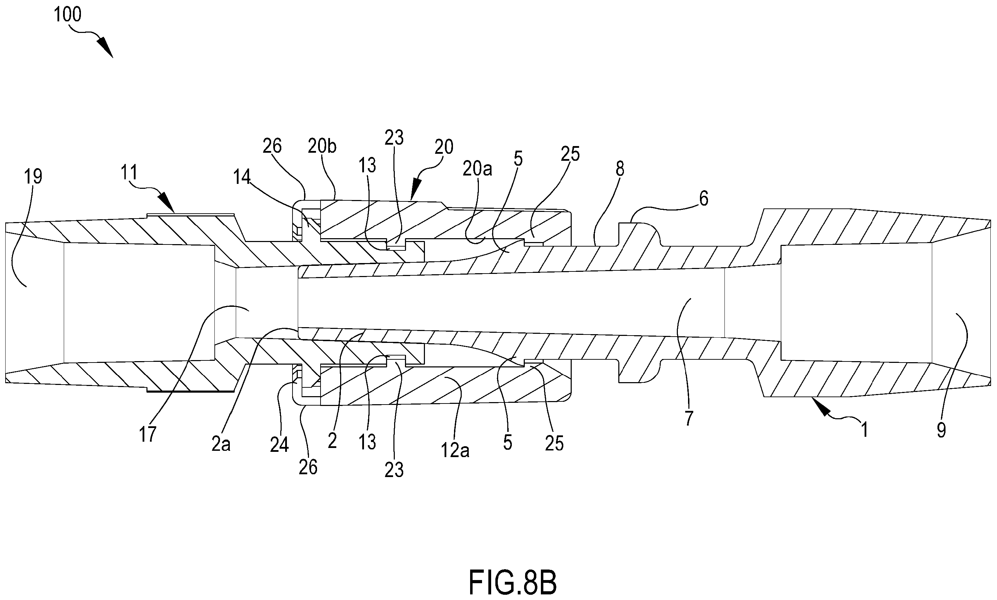

The second connector 11 may further comprise a hooking portion 13 arranged on an external lateral wall 11a of the main body of the second connector 11: in particular the hooking portion 13 may comprise a thread, preferably arranged in the proximity of the coupling portion 12 of the second connector 11. The threads of the hooking portion 13 preferably extend from the terminal end 12a of the second connector 11 and preferably emerge from the external lateral wall 11a of the main body of the second connector 11. In an alternative embodiment, the hooking portion 13 of the second connector 11 may comprise a respective groove or recess to define an undercut joint, such as a bayonet coupling (embodiment shown in figure 8). Said groove has a first tract extending substantially parallel to the axis of the second connector, and a second tract extending substantially perpendicular to the first tract. Alternatively said second tract may have arc shape with the concavity facing the terminal end 12a of the second connector 11.

The second connector 11 further comprises at least one locking projection or member 14 arranged on the same external lateral wall 11a of the main body of the second connector 11. In an embodiment, shown in figure 3, the at least one locking projection 14 is interposed between the hooking portion 13 and the connection port 19: similarly, the hooking portion 13 is interposed between the at least one locking projection 14 and the terminal end 12a of the second connector 11. The at least one locking projection 14 radially emerges outwardly from the main body of the second connector 11: in particular the at least one locking projection 14 comprises a plurality of socket teeth, wherein the socket teeth are wedge-shaped or have a saw tooth shape. The socket teeth are preferably arranged all around the tubular main body of the second connector 11, as shown in figure 3.

In the embodiment, the second connector 11 is a female connector and it is made in one single piece preferably in plastic material, such as PVC, MBS, PC or in metallic material, such as stainless steel. The second connector 11 may also comprise gripping means arranged on the external lateral wall 11a at the connection port 19: the gripping means are configured to allow an operator to grab firmly the second connector 11 especially when connection with the first connector 1 is required. The gripping means 15 preferably comprise tabs, in particular two tabs diametrically opposed, emerging radially outwardly from the main body of the second connector 11.

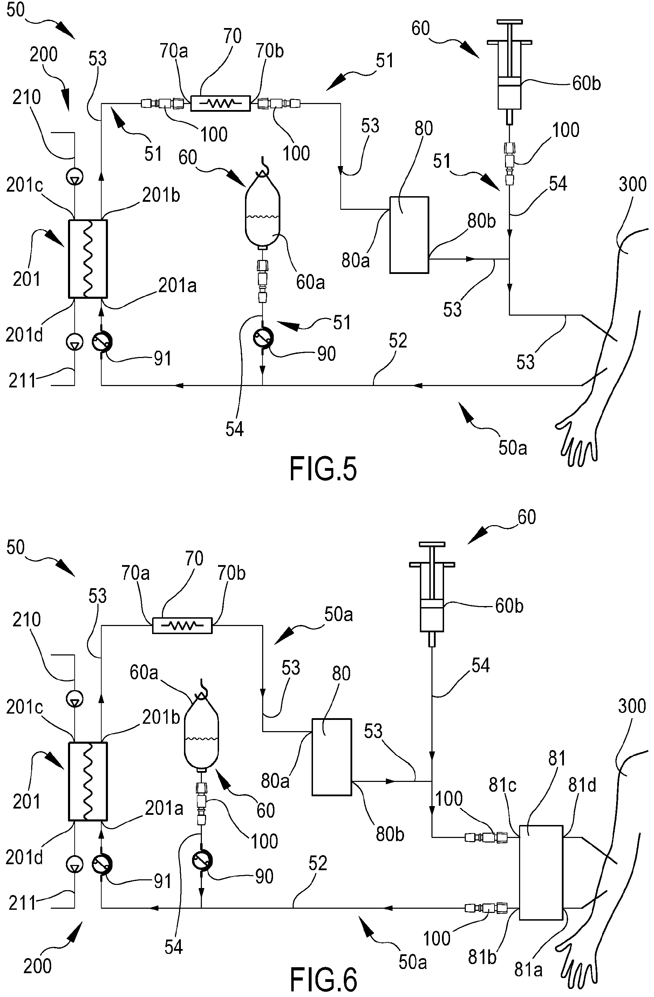

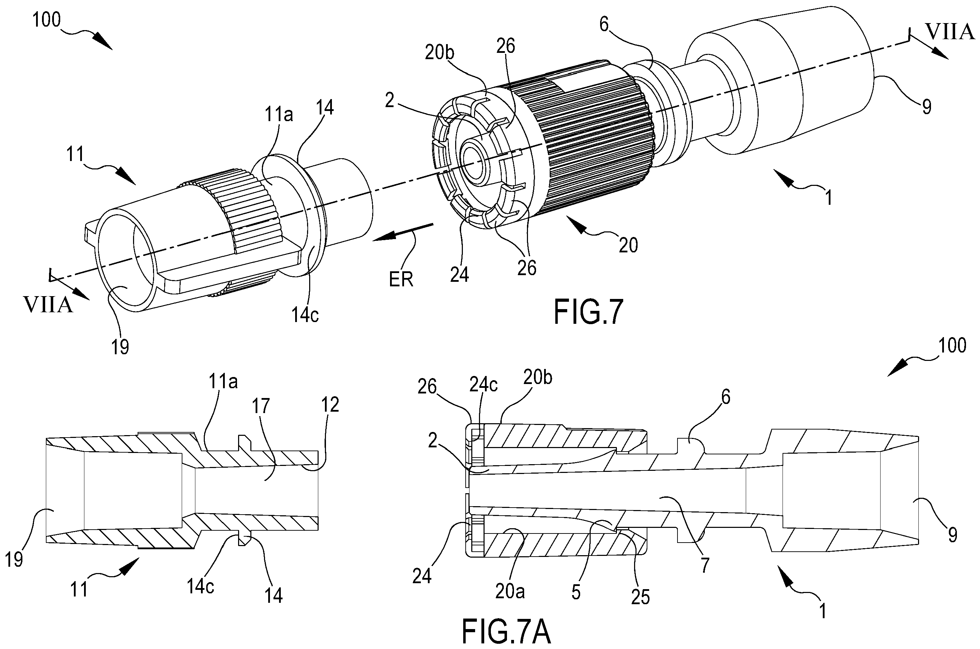

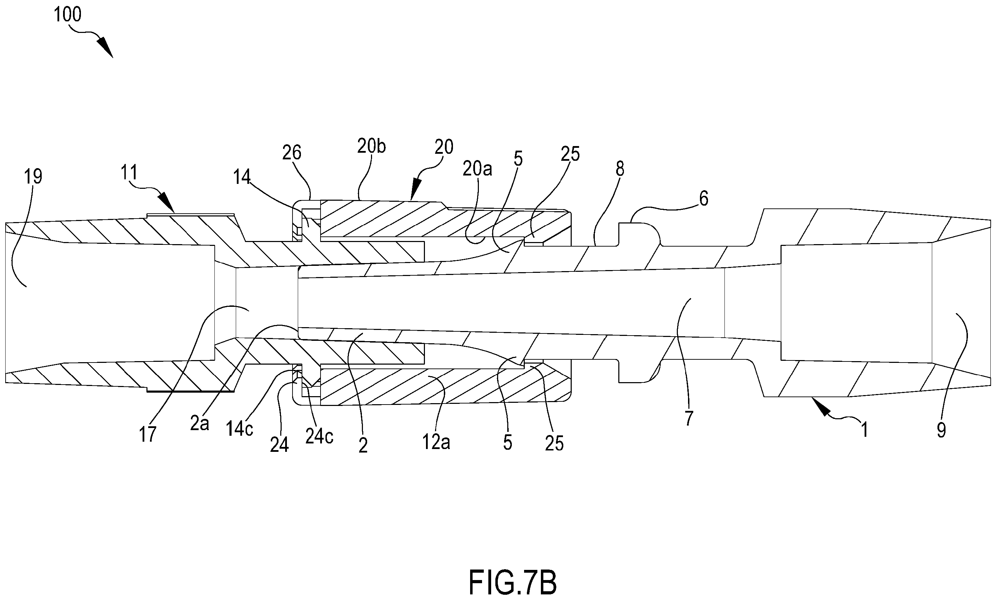

The connection system 100 further comprises a collar 20, shown in the exploded view of figure 3 and in the cross section view of figure 3A, presenting a main body having tubular shape: in particular the collar extends in length between a first opening 21 and a second opening 22, and the tubular main body comprises an external lateral wall 20b and an internal lateral wall 20a. In an embodiment not shown in the attached figures, the main body of the collar 20 may have polygonal shape, such as squared, rectangular or a combination of polygonal and tubular shapes. The collar 20 comprises an internal channel laterally limited by the first and the second openings 21, 22, wherein the opening 21 is configured to receive through insertion the second connector 11, in particular to receive the coupling portion 12, the hooking portion 13 and the at least one locking projection 14 of the second connector 11, while the second opening 22 allows the collar 20 to be mounted over, and coupled to, the first connector 1, as shown in figure 7. In more detail, the second opening 22 receives in insertion the first connector 1, so that the collar surrounds externally at least part of the first connector 1 and the main body of the collar 20 is coaxial with the first connector 1. In particular the collar 20 comprises, preferably at the second opening 22, a respective abutment portion 25 configured to contact the main abutment portion 5 of the first connector 1 when disposed in an abutment configuration, so that the collar 20 is axially constrained with respect to the first connector 1 at least along one axial direction. In particular the collar 20 is axially constrained towards the coupling portion 2 of the first connector 1, so that the collar 20 is prevented to axially move towards the coupling portion 2 of the first connector 1 beyond a certain limit. The abutment portion 25 of the collar 20, as shown in the section of figure 6, is arranged at the second opening 22 of the collar 20 and extends radially inwardly.

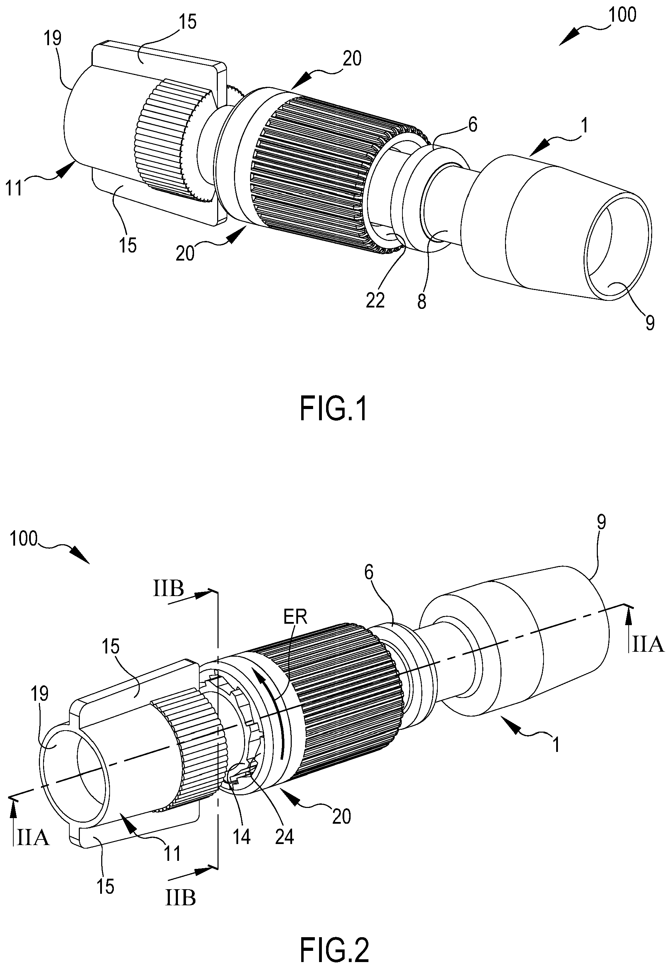

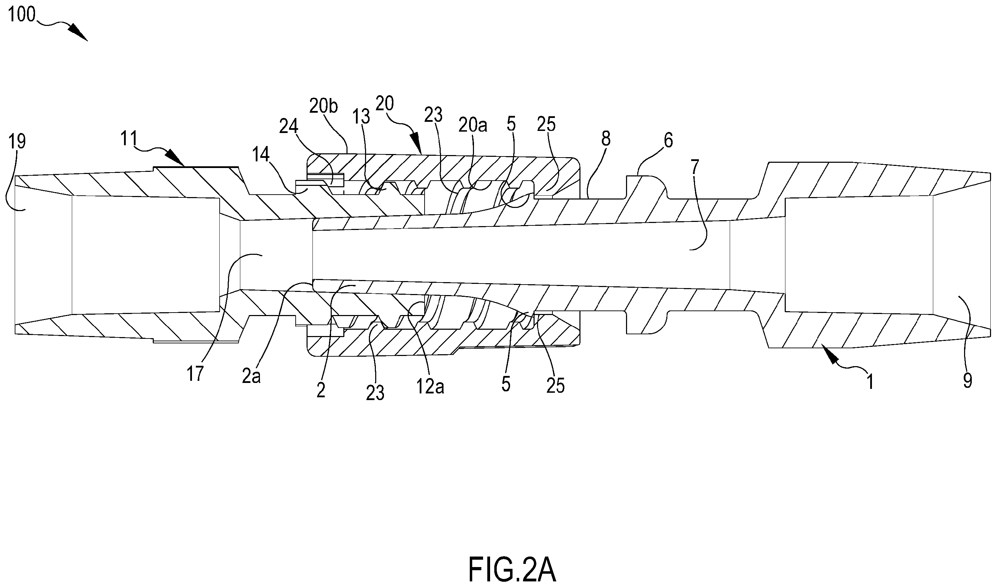

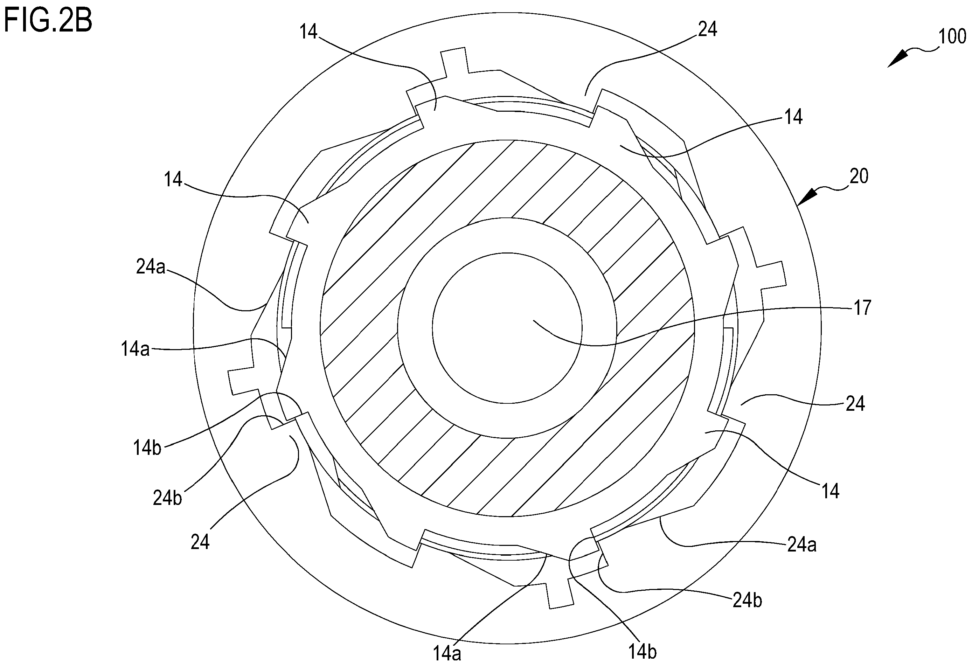

In the disclosed embodiment shown in figures 1 to 4, the first connector comprises both the main and the auxiliary abutment portions 5, 6, so that the abutment portion 25 of the collar is interposed between the main abutment portion 5 and the auxiliary abutment portion 6 of the first connector: therefore, the collar is a axially constrained to the first connector 1 through the main and auxiliary abutment portions 5, 6 of the first connector 1, so that an axial movement of the collar 20 with respect to the first connector 1 is substantially prevented. Alternatively or in addition, the collar 20 may be axially movable with respect to the first connector 1 within the axial gap defined between said main and auxiliary abutment portions 5, 6 of the first connector 1, and axially constrained to the first connector 1 out of this axial gap. In particular, the collar 20 is axially movable with respect to the first connector 1 of an amount proportional to the distance between the main and the auxiliary abutment portions 5, 6 of the first connector 1. The axial movement is defined along the fluid flow axis of the first connector 1 and/or of the second connector 11. Anyhow, if the first connector comprises the main and the auxiliary abutment portions 5, 6, the abutment portion 25 of the collar 20 is arranged at, and in particular surrounds, the recess 8 of the first connector 1. In this specific embodiment, the second opening 22 has an internal diameter lower than an external diameter of the main abutment portion 5 of the first connector 1, preventing in this way the axial movement. In other words, the first connector 1 and the collar, although they are distinct bodies both made in a single piece, are axially coupled each other, being the collar 20 movable by rotation with respect to the first connector 1. In an alternative embodiment not shown in the attached figures, the collar 20 and the first connector 1 are made in one single piece. In an embodiment, the collar 20 has a length, measured along an axis of its tubular main body, comprised between 8 mm and 25 mm, and a diameter comprised between 7 mm and 15 mm. The collar 20 may further comprise a respective hooking portion 23 configured to be engaged with the hooking portion 13 of the second connector 11 through an engagement rotation ER along an engagement direction (see figure 2) defining an engaged configuration shown in figures 1 and 2, so that when the connection system 100 is disposed in this engaged configuration the hooking portion 23 of the collar 20 and the hooking portion 13 of the second connector 11 prevent axial removal of the collar 20 from the second connector 11. In an embodiment, the hooking portion 23 of the collar comprises a thread configured to engage the thread of the second connector 11 through this engagement rotation. In an embodiment, the hooking portion 23 is arranged on the internal lateral wall 20a of the collar 20. In other words, in this engaged configuration, the collar 20 is screwed over the second connector 11 through the respective threads, defining an axial constraint between the collar 20 and the second connector 11.

In an alternative embodiment, the hooking portion 23 of the collar 20 comprises an undercut joint (see figure 8) and the hooking portion 13 of the second connector 11 comprises the respective undercut joint, wherein the undercut joint of the collar 20 is configured to axially engage the undercut joint of the second connector 11 defining a bayonet coupling for example through the same engagement rotation ER, so that an axial movement, in particular an axial separation, of the first and second connectors 1, 11 is prevented: this axial movement or axial separation is considered along the fluid flow axis of the first or second connectors 1, 11. In this alternative embodiment, the engagement rotation is comprised between 10° and 180°, in particular comprised between 15° and 95°.

Since the collar 20 is axially coupled to the first connector 1, when the connection system 100 is disposed in this engaged configuration the first connector 1 is axially coupled to the second connector 11, so that disconnection of the first and second connectors 1, 11 is prevented. In particular, at an end stage of the engagement rotation, the hooking portions 13, 23 respectively of the second connector 11 and of the collar 20 contribute to tighten the connection and to prevent disconnection between the coupling portion 2 of the first connector 1 and the coupling portion 12 of the second connector 11. During an engagement rotation ER wherein the collar 20 rotates to determine the engagement rotation, the coupling portion 2 of the first connector 1 is allowed to stay aligned with the coupling portion 12 of the second connector 11 due to the fact that the collar 20 is freely movable by rotation with respect to the first connector 1: once the connection between the coupling portion 2 of the first connector 1 and the coupling portion 12 of the second connector 11 is established, the collar 20 is still freely movable by rotation with respect to the first and second connectors 1, 11, so that the subsequent engagement rotation ER does not determined friction between the coupling portion 2 of the first connector 1 and the coupling portion 12 of the second connector 11. In other words and according to an embodiment, during the engagement rotation the first and the second connectors 1, 11 may be aligned each other without reciprocal rotation, while the collar 20 rotates with respect both the first and second connectors 1, 11 to determine engagement of the hooking portions 13, 23.

The hooking portion 23 of the collar 20 may comprise, as shown in figure 8, a protrusion radially extending inwardly and configured to insert into the groove of the hooking portion 13 of the second connector. The collar 20 further comprises a respective at least one locking projection 24 engageable to the at least one locking projection 14 of the second connector 11 through the engagement rotation ER of the collar 20 with respect to the second connector 11 along the engagement direction. The connection system 100 may be disposed in a coupled configuration, wherein the at least one locking projection 24 of the collar 20 cooperates with the at least one locking projection 14 of the second connector 11, preventing a disengagement rotation of the collar 20 with respect to the second connector 11: this disengagement rotation is a rotation in a opposite direction with respect to the engagement rotation. The coupled configuration is defined at least at an end stage of the engagement rotation between the collar 20 and the second connector 11, meaning that when the engagement rotation is complete, a disengagement rotation of the collar 20 with respect to the first connector 1 is prevented. In addition, the coupled configuration may be defined at an intermediate or initial stage of the engagement rotation ER: this means that, as soon as the hooking portion 23 of the collar 20 engages the hooking portion 13 of the second connector 11, the locking projections 14, 24, respectively of the second connector 11 and of the collar 20, also cooperate each other, preventing the disengagement rotation.

In an embodiment, this locking projections 24 are arranged on the internal lateral wall 20a of the collar, so that the locking projection 24 and the hooking portion 23 of the collar 20 are arranged side by side. In particular, the at least one locking projection 24 of the collar may comprise a plurality of socket teeth, wherein the socket teeth are wedge-shaped or have a saw tooth shape. The at least one locking projection 14 radially emerges inwardly from the internal lateral wall 20a of the collar, as shown in figure 3. When the socket teeth of the collar 20 face, and in particular contact, the socket teeth of the second connector 11, the engagement rotation is allowed and the disengagement rotation is prevented.

The socket teeth of both the second connector 11 and the collar 20 are arranged circumferentially all around the external lateral wall 11a of the second connector 11 and circumferentially all around the internal lateral wall 20a of the collar 20. The socket teeth radially define recesses and protrusions alternately, so that the second connector 11 defines a maximum external diameter of encumbrance measured at the protrusions of the socket teeth, and wherein the collar 20 defines a minimum internal diameter measured at the protrusions of the socket teeth of the collar 20: this maximum external diameter of encumbrance is higher than this minimum internal diameter so that, when the socket teeth of the second connector 11 are faced to or in contact with the socket teeth of the collar 20, the protrusions of the socket teeth of the second connector 11 are placed into the recesses of the socket teeth of the collar 20 and vice versa. This is clearly shown in the cross section view of figure 2B, wherein the saw tooth shape of the locking projections allows the engagement rotation and prevents the disengagement rotation.

In an embodiment, the socket teeth of the second connector 11 and of the collar 20 comprise each a respective slide surface 14a, 24a (figure 2B) tilted with respect to the external surface of the second connector 11 and to the internal lateral wall 20a of the collar 20: preferably this slide surface 24a is tilted by an angle lower than 60°. Each of the socket teeth of the second connector 11 and of the collar 20 further comprise respective lock surfaces 14b, 24b which are substantially perpendicular with respect to the external surface of the second connector 11 and to the internal lateral wall 20a of the collar 20: the slide surface 14a, 24a and the lock surface 14b, 24b of each socket tooth are connected together at a vertex defining the protrusion. In other words, the slide surface 14a, 24a and the lock surface 14b, 24b, of one socket tooth define a triangle shaped tooth, wherein these teeth are radially arranged over the internal lateral wall 20a of the collar 20 and over the external lateral wall 11a of the second connector 11. The socket teeth of the collar 20 are oriented in an opposite direction with respect to the socket teeth of the second connector 11, so that the lock surfaces 14b of the socket teeth of the second connector 11 may be faced to the lock surfaces 24b of the collar 20. Each slide surface 14a of a socket tooth of the second connector 11 is configured to cooperate or contact with a respective slide surface 24a of a socket tooth of the collar 20, so that the engagement rotation is allowed: on the other hand, each lock surface 14b of a socket tooth of the second connector 11 is configured to contact, when the connection system 100 is disposed in the coupled configuration, with a respective lock surface 24b of a socket tooth of the collar 20, preventing therefore the disengagement rotation: in particular, when the connection system 100 is disposed in the coupled configuration, each lock surface 14b of a socket tooth of the second connector 11 abuts against a respective lock surface 24b of a socket tooth of the collar 20.

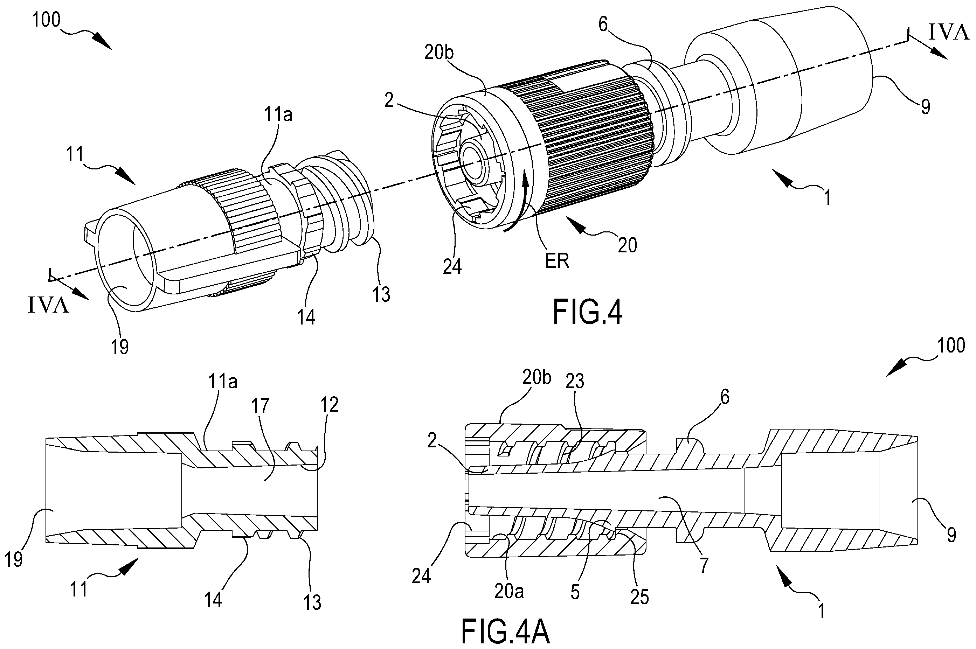

In an embodiment shown in figures 3, 3A, 4 and 4A, the hooking portion 13 of the second connector 11 is interposed between the at least one locking projection 14 and the terminal portion 12a of the coupling portion 12, and the at least one locking projection 24 of the collar 20 is arranged at an inlet of the first opening 21 so that the hooking portion 23 of the collar 20 is interposed between the at least one locking projection 24 and the second opening 22.

In an alternative embodiment not shown in the attached figures, the at least one locking projection 14 of the second connector 11 is interposed between the hooking portion 13 and the terminal portion 12a of the coupling portion 12, and the hooking portion 23 of the collar 20 is arranged at the inlet of the first opening 21 so that the at least one locking projection 24 of the collar 20 is interposed between the hooking portion 23 and the second opening 22 of the collar 20. The connection system 100 is configurable in an unlocked condition, wherein the collar 20 is movable by rotation with respect to the first connector 1 and the at least one locking projection 24 of the collar 20 and the at least one locking projection 14 of the second connector 11 are in an uncoupled configuration wherein the locking projections do not cooperate each other. The unlocked condition is shown in figure 4, wherein the first and the second connectors 1, 11 are separated: when the first and the second connectors 1, 11 are separated, the collar 20 is anyhow axially constrained to the first connector through the main and auxiliary abutment portions 5, 6 of the first connector 1 cooperating with the abutment portion 25 of the collar. In addition, the unlocked condition may also be defined when the hooking portion 13 of the second connector 11 is engaged with the hooking portion 23 of the collar 20, which defines the engaged configuration: this means that, when an engagement rotation is at an initial or intermediate stage, the at least one locking projection 24 of the collar 20 and the at least one locking projection 14 of the second connector 11 do not cooperate yet, allowing disengagement rotation of the collar 20 with respect to the first connector 1. The connection system 100 is further configurable in a locked condition, shown in figures 1, 2, and in the section view of figure 2A, wherein the at least one locking projection 24 of the collar 20 and the at least one locking projection 14 of the second connector 11 are in the coupled configuration, and the hooking portion 23 of the collar 20 and the hooking portion 13 of the second connector 11 are in the engaged configuration. In this locked condition, the collar 20 is irremovably engaged to the second connector 11 through said engagement rotation: in particular, the at least one locking projection 14 and the hooking portion 13 of the second connector 11 respectively cooperate with the at least one locking projection 24 and the hooking portion 23 of the collar 20 preventing disengagement of the first connector 1 from the second connector 11. Moreover, when the connection system 100 is disposed in the locked condition, the coupling portion 2 of the first connector 1 is connected to and/or inserted into the coupling portion 12 of the second connector 11, so that fluid tightness between the first and the second connector 1, 11 is established. Moreover, in this locked condition, the abutment portion 25 of the collar 20 abuts against the main abutment portion 5 of the first connector 1, preventing the axial movement, and therefore separation, of the first connector with respect to the collar.

In an embodiment, the collar 20, when engaged to the second connector 11 by the respective threads, may present a slight axial movement with respect to the second connector 11, especially at an initial or intermediate stage of the engagement rotation: this may happen if the width of the thread wire of the second connector 11, is lower than the thread pitch of the collar 20, defining a gap in between, which leads to an axial movement of the collar with respect to the second connector. In this case, the locking portions 14 and 24 respectively of the second connector 11 and of the collar 20 should have a width, measured parallel to the fluid flow axis, higher than this gap, in order to prevent decoupling. In the shown embodiment, when the connection system 100 is in the locked condition and/or in the engaged configuration, the fluid flow axis of the first connector 1 coincides with the fluid flow axis of the second connector 11, and the collar is coaxial with this fluid flow axis.

Figures 8, 8A, 8B and 7, 7A and 7B show further embodiments of the non-removable connection system wherein the second connector 11 comprises the at least one locking projection 24 and the collar comprises the respective at least one locking projection 14. According to the embodiments shown in figures 7, 7A and 7B, no hooking portions, such as threads or bayonet couplings, are provided on the second connector and on the collar.

The locking projections 14 of the second connector 11 and the locking projections 24 of the collar 20 comprise at least one protrusion respectively extending radially outwardly and inwardly. The protrusions of the collar 20 comprise an abutment portion 24c facing the inner volume of the collar 20, while the protrusions of the second connector 11 comprise an abutment portion 14c facing opposite with respect to the fluid coupling portion 12 of the second connector 11. The abutment portion 24c of the protrusions 24 of the collar 20 are configured to abut against the abutment portion 14c of the protrusions 14 of the second connector 11, as shown in the section of figures 7B and 8B, when the connection system is arranged in the locked condition in order to prevent axial disengagement of the collar from the second connector. The collar 20 and the second connector 11, according to the embodiment of figures 8, 8A and 8B, further comprises the hooking portions 23 and 13: in particular figure 8 shows a hooking portion having bayonet coupling. Anyhow, a hooking portion comprising respective threads may also be provided according to this embodiment.

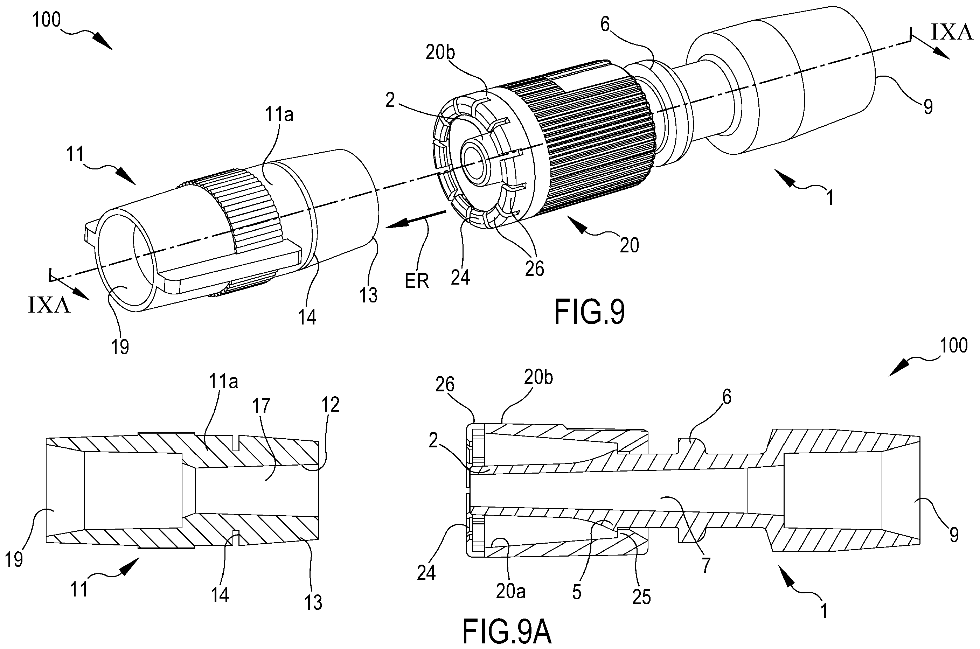

According to the previously embodiments, the locking projections 14, 24 of the second connector and collar comprise protrusions, such as wedge shaped socket teeth: anyhow, as shown in figure 9 and 9A according to a further embodiment, the second connector comprises a locking member 14 having at least one recess, optionally an aperture, and the collar 20 comprises a locking member 24 having at least one protrusion: the protrusions of the collar 20 are configured, in the locked condition, to engage the recess of the second connector 11: in particular the protrusions of the collar 20 are configured to enter at least partially into said recess to define an axial engagement. Therefore, the embodiments shown in figures 1-4A define the coupled configuration and the locked condition by the engagement rotation ER of the collar 20 with respect to the second connector 11: contrary, the embodiments of figures 7 to 9A define the coupled configuration and the locked condition by an axial engagement displacement along the axis of the first and second connector (namely the axis of the connection system). In other terms, the connection system is configurable, through the axial engagement displacement, in a locked condition, wherein the locking projections or members 24 of the collar 20 and the locking projections or members 14 of the second connector 11 are in the coupled configuration, wherein in this locked condition the collar 20 is irremovably engaged to the second connector 11 through the axial engagement displacement, and the locking projections or members 14 of the second connector 11 cooperate with the locking projections or members 24 to prevent disengagement, in particular axial disengagement, of the first connector 1 from the second connector 11. The axial engagement displacement is a displacement wherein the collar and the second connector are approached each other and axially coupled. According to the embodiments of figures 7, 7A, 7B and 9, the collar 20 may freely rotate with respect to the second connector 11 when arranged in the locked condition, preventing at the same time axial disengagement between the collar and the second connector, and therefore preventing axial disengagement between the first and the second connector. Notably, the coupling portions 2 and 12 of the first and second connectors 1, 11 abut each other to define a fluid tight coupling when the connection system is arranged in the locked condition.

According to the above description, it is to be understood that the invention is not supposed to be limited to the disclosed embodiments, but on the contrary, is intended to cover various modifications and arrangements included within the scope of the appended claims.

### Disposable set 50

The invention also refers to a disposable set 50, shown in figures 5 and 6, for a blood treatment apparatus 200, such as a dialysis machine. The disposable set 50 comprises a plurality of fluid lines 51, such as a blood circuit 50a which includes an access line 52 configured to be connected to a patient 300 and to an inlet port 201a of a blood treatment unit 201, such as a hemofilter, an ultrafilter, a hemodiafilter, a dialyzer, a plasmafilter and the like. The blood circuit 50a also comprises a return line 53 configured to be connected to the patient 300 and to an outlet port 201b of the same blood treatment unit 201. In this configuration, blood is supposed to flow from the patient along the access line 52, through the blood treatment unit 201, and towards the return line 53, as shown by the arrows depicted on the fluid lines of figures 5 and 6. The disposable set 50 may also comprise one or more infusion lines 54 connected to the blood circuit 52 and/or configured to be connected to the patient 300. The infusion line 54 may comprise a container 60 connected to one end of the infusion line and wherein the other end of the infusion line 54 is connected to the blood circuit or to the patient. This container 60 is configured for housing an infusion fluid and is made, for example, of plastic material for medical applications: in particular the container 60 may be a citrate solution container 60a containing a citrate concentrated solution, or calcium solution container 60b containing a calcium concentrated solution, or a substitute fluid solution to be injected into the blood circuit during treatment. Figures 5 and 6 shows a (pre-) infusion line 54 arranged upstream with respect to the blood treatment unit 201 and connected to a citrate solution container 60a. The infusion line 54 is also placed upstream a blood pump 91 to provide regional anticoagulation to the extracorporeal blood. At least one pump 90 may be associated to the infusion line 54 and configured to determine fluid flow from the container 60 towards the other end of the infusion line 54, for example towards the access line 52 or to the return line 53. In more detail, the pumps 90 and 91 are peristaltic pumps. Other infusion lines may be included in the circuit and are not represented. For example a pre-infusion line injecting substitution fluid upstream the blood treatment unit 201 and downstream the blood pump 91 may be included, as well as a post-infusion line injecting a substitute solution into the return line 53, for example into the venous bubble trap (not shown). Figures 5 and 6 also show an additional infusion line 54 arranged downstream with respect to the to the blood treatment unit 201 and connected to the calcium solution container 60a, for example a syringe configured for housing a calcium solution. This infusion line is intended to reestablish the ion balance in a regional anticoagulation procedure. The blood pump 91 is associated to the access line 52 and/or to the return line 53 to determine blood flow through the treatment unit 201. The disposable set 50 may also comprise a monitoring line 55, not shown in the attached figures, connected to the blood circuit 50a or to any of the infusion lines 54, and configured e.g., to be connected to a pressure or to temperature gauge for parameter detection or to be used as a site access for blood sampling. The disposable set 50 may also comprise a warmer accessory 70, such as a bag or a line and schematically shown in figures 5 and 6, connected to the blood circuit 50a (e.g. downstream the treatment unit 201) and configured to warm up the blood before return to the patient 300. In particular the warmer accessory 70 is connected in series to the return line 53, so that an inlet 70a of the warmer accessory 70 is connected to an upstream tract of the return line 53, and an outlet 70b of the warmer accessory 70 is connected to a downstream tract of the return line 53. The disposable set 50 further comprises at least one connection system 100 as previously described, which may be connected to the access line 52, the return line 53, to the infusion line 54 and the monitoring line 55. In figure 5 the connection system 100 connects the (calcium) infusion line 54 to the blood circuit; alternatively or in addition the connection system may be used to connect the citrate solution container 60a to the pre blood pump infusion line 54. Moreover, the connections system 100 may connect the inlet 70a and the outlet 70b of the warmer accessory 70 to the return line 53, in order to prevent any undesired disconnection. The disposable set 50 also comprises the blood treatment unit 201 having a blood inlet 201a connected to the access line 52, a blood outlet 201b connected to the return line 53, a fluid inlet 201c connected to a fluid delivery line 210 configured to be connected to a source of fresh treatment fluid (not shown in the attached figures), and a fluid outlet 201d connected to a fluid output line 211 configured to be attached to an exhaust unit, such as a discharge bag. In an embodiment not shown in the attached figures, the connection system 100 may also be used to connected the blood inlet 201a of the blood treatment unit 201 to the access line 52, the blood outlet 201b to the return line 53, the fluid inlet 201c to the fluid delivery line 210 and the fluid outlet 201d to the fluid output line 211 (if proper or necessary). The disposable set 50 may also comprise a disposable accessory 80, such as a gas exchanger, an oxygenator or an auxiliary fluid arrangement, connected to the blood line 50a, in particular a gas exchanger 80 may be connected in series to the return line 53. The gas exchanger 80 is configured to remove CO₂ from the blood before returning it to the patient 300. The gas exchanger comprises a blood inlet 80a and a blood outlet 80b for connection to the return line 53: the connection system 100, in an embodiment not shown in the attached figures, may further be used for connecting the blood inlet 80a of the gas exchanger 80 to an upstream tract of the return line 53, and the blood outlet 80b to a downstream tract of the return line 53. The disposable set may also comprise an add-on accessory 81 connected to the blood circuit 50a through the connection system 100 as shown in figure 6. The add-on accessory 81 is interposed between the patient (in particular the patient vascular access) and the access and return lines of the blood circuit; the add-on accessory 81 comprises an arterial blood inlet 81a configured to be connected to an arterial access of the patient 300, an arterial blood outlet 81b connected to the access line 52 of the disposable set 50, a venous blood inlet 81c connected to the return line 53 of the disposable set 50, and a venous blood outlet 81d configured to be connected to a venous access of the patient 300. The connection system may be interposed in connection between the arterial blood outlet 81b and the access line 52, and between the venous blood inlet 81c and the return line 53. Obviously, the disposable set may include several additional elements not represented/described since well-known to the skilled person, such as deaeration chambers, pressure monitoring components, infusion or sampling sites, bubble detectors, venous and arterial clamps to stop blood circulation, etc.... The disposable set may be a set for acute or chronic treatments and may assume different configurations with respect to the shown exemplary set. While the invention has been described in connection with what is presently considered to be the most practical and preferred embodiment, it is to be understood that the invention is not to be limited to the disclosed embodiment, but on the contrary, is intended to cover various modifications and arrangements included within the scope of the appended claims.

## Claims

1. A non-removable connection system (100) for a medical apparatus, said connection system (100) comprising:
- a first connector (1) comprising a fluid coupling portion (2), a main abutment portion (5) arranged on an external lateral wall of the first connector (1) and defining a protrusion radially extending outward from the first connector (1), the first connector (1) being a Luer type male connector;
- a second connector (11) comprising a respective fluid coupling portion (12) connectable, in a fluid tight manner, to the fluid coupling portion (2) of the first connector (1), so that a fluid is able to internally flow between the first and the second connector (1, 11), the second connector (11) further comprising at least one locking member (14), the second connector (11) being a Luer type female connector;
- a collar (20) coupled to the first connector (1) and comprising:
a respective abutment portion (25) configured to contact the main abutment portion (5) of the first male connector (1) when disposed in an abutment configuration, so that the collar (20) is axially constrained with respect to the first connector (1) at least along one axial direction;
a respective at least one locking member (24) engageable to the at least one locking member (14) of the second connector (11) through an axial engagement displacement along an engagement direction (ED), in a coupled configuration the at least one locking member (24) of the collar (20) and the at least one locking member (14) of the second connector (11) preventing an axial disengagement displacement of the collar (20) with respect to the second connector (11), said axial disengagement displacement being a displacement in an opposite direction with respect to said axial engagement displacement, the collar (20) comprising a main body having tubular shape extending between a first opening (21) configured to receive the second connector (11), and a second opening (22) receiving by insertion the first connector (1), the collar (20) comprising a plurality of fingers (26) arranged at the first opening (21) angularly distributed about the axis of the collar (20), said fingers (26) carrying the at least one locking member (24) of the collar (20);
and wherein the connection system (100) is configurable, through the axial engagement displacement, from:
- an unlocked condition, wherein the at least one locking member (24) of the collar (20) and the at least one locking member (14) of the second connector (11) are in an uncoupled configuration; to
- a locked condition wherein the at least one locking member (24) of the collar (20) and the at least one locking member (14) of the second connector (11) are in the coupled configuration, in said locked condition the collar (20) being irremovably engaged to the second connector (11) through said axial engagement displacement, and the at least one locking member (14) of the second connector (11) cooperating with the at least one locking member (24) preventing disengagement of the first connector (1) from the second connector (11),
and wherein:
- the at least one locking member (14) of the second connector comprises at least one recess, and the at least one locking member (24) of the collar (20) comprises at least one protrusion, the at least one protrusion of the collar (20) being configured in the locked condition to engage the recess of the second connector (11) to define a non removable axial engagement, or
- the at least one locking member (14) of the second connector (11) comprises at least one protrusion, and the at least one locking member (24) of the collar (20) comprises at least one recess, the at least one protrusion of the second connector (11) being configured in the locked condition to engage the recess of the collar (20) to define a non removable axial engagement.

2. The connection system of the preceding claim, wherein the axial engagement displacement is a displacement directed substantially parallel to the axis of the first and second connectors (1, 11), in particular said axial engagement displacement being a displacement directed along the axis of the first and second connectors (1, 11), in particular along the axis of the connection system.

3. The connection system of any one of the preceding claims 1 and 2, wherein a switch from the locked condition to the unlocked condition is prevented by the at least one locking projection or locking member (14, 24).

4. The connection system of any one of the preceding claims 1-2, wherein:
- the at least one recess of the at least one locking member (14) of the second connector (11) is an aperture, and the at least one locking member (24) of the collar (20) comprises the at least one protrusion, the at least one protrusion of the collar (20) being configured in the locked condition to enter the recess of the second connector (11) to define an non removable axial engagement, in particular said protrusion radially extending inwardly, or
- the at least one locking member (14) of the second connector (11) comprises the at least one protrusion, and the at least one recess of the at least one locking member (24) of the collar (20) is an aperture, the at least one protrusion of the second connector (11) being configured in the locked condition to enter the recess of the collar (20) to define an non removable axial engagement, in particular said protrusion radially extending outwardly.

5. The connection system of any one of the preceding claims , wherein the one or more fingers (26) extend substantially parallel to the axis of the collar (20).

6. The connection system of any one of the preceding claims, wherein said fingers (26) of the collar (20) carries the protrusions of the collar (20).

7. The connection system of any one of the preceding claims 1-6, the locking projections or member (24) of the collar define a minimum inner diameter smaller than a maximum outer diameter defined by the locking projections or members (14) of the second connector (11), wherein said fingers (26) of the collar (20) are configured to radially flex outwardly during coupling of the locking projections (14) of the second connector with the locking projections (24) of the collar (20).

8. The connection system of any one of the preceding claims, wherein the collar 20 freely rotates with respect to the second connector 11 when arranged in the locked condition, preventing at the same time axial disengagement between the collar and the second connector, and therefore preventing axial disengagement between the first and the second connector.

9. The connection system of the preceding claims, wherein the second connector (11) comprises a hooking portion (13), and the collar comprises a respective hooking portion (23) engageable to the hooking portion (13) of the second connector (11) through an engagement rotation along an engagement direction (ER), in a engaged configuration the hooking portion (23) of the collar (20) and the hooking portion (13) of the second connector (11) preventing axial removal of the collar (20) from the second connector (11).

10. The connection system of the preceding claim, wherein the hooking portion (23) of the collar (20) and the hooking portion (13) of the second connector both comprise respective threads configured to engage each other to define the engaged configuration; or respective undercut joints configured to engage each other as a bayonet coupling to define the engaged configuration.

11. The connection system of the preceding claim, wherein the hooking portion (13) of the second connector (11) comprises a groove to define the undercut joint, in particular a bayonet coupling,
and wherein said groove has a first tract extending substantially parallel to the axis of the second connector, and
- a second tract extending substantially perpendicular to the first tract, or
- said second tract being arc shaped with concavity facing a terminal portion (12a) of the second connector (11), the fluid coupling portion (12) of the second connector (11) including said terminal portion (12a) defining an internal channel (17) for fluid transport.

12. The connection system of any one of the preceding claims, wherein said axial disengagement displacement is a linear displacement in an opposite direction with respect to said axial engagement displacement.

13. Disposable set (50) for a blood treatment apparatus, said disposable comprising:
- a blood circuit (50a) including:
• an access line (52) configured to be connected to a patient (300) and to an inlet port (201a) of a blood treatment unit (201);
• a return line (53) configured to be connected to the patient (300) and to an outlet port (201b) of the blood treatment unit (201);
- optionally an infusion line (54) connected to the blood circuit (52) and/or configured to be connected to the patient (300);
- optionally a monitoring line (55) connected to the blood circuit (50a) or to the infusion line (54);
- at least one connection system (100) according to any one of the claims from 1 to 12 connected to at least one between the access line (52), the return line (53), the infusion line (54) and the monitoring line (55).

## Patentansprüche

1. Nicht entfernbares Verbindungssystem (100) für eine medizinische Vorrichtung, wobei das Verbindungssystem (100) Folgendes umfasst:
- einen ersten Verbinder (1), der einen Fluidkopplungsabschnitt (2) und einen Hauptanlageabschnitt (5) umfasst, der an einer äußeren Seitenwand des ersten Verbinders (1) angeordnet ist und einen Vorsprung definiert, der sich von dem ersten Verbinder (1) radial nach außen erstreckt, wobei der erste Verbinder (1) ein Luer-Stecker ist,
- einen zweiten Verbinder (11), der einen jeweiligen Fluidkopplungsabschnitt (12) umfasst, der fluiddicht mit dem Fluidkopplungsabschnitt (2) des ersten Verbinders (1) verbindbar ist, so dass ein Fluid intern zwischen dem ersten und dem zweiten Verbinder (1, 11) strömen kann, wobei der zweite Verbinder (11) ferner mindestens ein Verriegelungsglied (14) umfasst, wobei der zweite Verbinder (11) eine Luer-Buchse ist,
- einen Bund (20), der an den ersten Verbinder (1) gekoppelt ist und Folgendes umfasst:
einen jeweiligen Anlageabschnitt (25), der dazu ausgestaltet ist, den Hauptanlageabschnitt (5) des ersten männlichen Verbinders (1) zu kontaktieren, wenn er in einer Anlagekonfiguration angeordnet ist, so dass der Bund (20) in Bezug auf den ersten Verbinder (1) mindestens entlang einer axialen Richtung axial festgehalten ist,
ein jeweiliges mindestens ein Verriegelungsglied (24), das mit dem mindestens einen Verriegelungsglied (14) des zweiten Verbinders (11) durch eine axiale Eingriffsverschiebung entlang einer Eingriffsrichtung (ED) in Eingriff bringbar ist, wobei das mindestens eine Verriegelungsglied (24) des Bunds (20) und das mindestens eine Verriegelungsglied (14) des zweiten Verbinders (11) in einer gekoppelten Konfiguration eine axiale Ausrückverschiebung des Bunds (20) in Bezug auf den zweiten Verbinder (11) verhindern, wobei die axiale Ausrückverschiebung eine Verschiebung in einer entgegengesetzten Richtung in Bezug auf die axiale Eingriffsverschiebung ist, wobei der Bund (20) einen Hauptkörper mit Röhrenform umfasst, der sich zwischen einer zur Aufnahme des zweiten Verbinders (11) ausgestalteten ersten Öffnung (21) und einer zweiten Öffnung (22) erstreckt, die den ersten Verbinder (1) zur Einführen aufnimmt, wobei der Bund (20) eine Vielzahl von Fingern (26) umfasst, die an der ersten Öffnung (21) angeordnet und winkelmäßig um die Achse des Bunds (20) verteilt sind, wobei die Finger (26) das mindestens eine Verriegelungsglied (24) des Bunds (20) tragen,
und wobei das Verbindungssystem (100) durch die axiale Eingriffsverschiebung aus:
- einem entriegelten Zustand, in dem das mindestens eine Verriegelungsglied (24) des Bunds (20) und das mindestens eine Verriegelungsglied (14) des zweiten Verbinders (11) in einer entkoppelten Konfiguration sind, in
- einen verriegelten Zustand konfigurierbar ist, in dem sich das mindestens eine Verriegelungsglied (24) des Bunds (20) und das mindestens eine Verriegelungsglied (14) des zweiten Verbinders (11) in der gekoppelten Konfiguration befinden, wobei der Bund (20) in dem verriegelten Zustand durch die axiale Eingriffsverschiebung unlösbar mit dem zweiten Verbinder (11) in Eingriff steht und wobei das mindestens eine Verriegelungsglied (14) des zweiten Verbinders (11) mit dem mindestens einen Verriegelungsglied (24) zusammenwirkt, um ein Ausrücken des ersten Verbinders (1) aus dem zweiten Verbinder (11) zu verhindern,
und wobei:
- das mindestens eine Verriegelungsglied (14) des zweiten Verbinders mindestens eine Aussparung umfasst und das mindestens eine Verriegelungsglied (24) des Bunds (20) mindestens einen Vorsprung umfasst, wobei der mindestens eine Vorsprung des Bunds (20) dazu ausgestaltet ist, in dem verriegelten Zustand die Aussparung des zweiten Verbinders (11) in Eingriff zu nehmen, um einen nicht entfernbaren axialen Eingriff zu definieren, oder
- das mindestens eine Verriegelungsglied (14) des zweiten Verbinders (11) mindestens eine Vorsprung umfasst und das mindestens eine Verriegelungsglied (24) des Bunds (20) mindestens eine Aussparung umfasst, wobei der mindestens eine Vorsprung des zweiten Verbinders (11) dazu ausgestaltet ist, in dem verriegelten Zustand die Aussparung des Bunds (20) in Eingriff zu nehmen, um einen nicht entfernbaren axialen Eingriff zu definieren.

2. Verbindungssystem nach dem vorhergehenden Anspruch, wobei die axiale Eingriffsverschiebung eine im Wesentlichen parallel zu der Achse des ersten und des zweiten Verbinders (1, 11) gerichtete Verschiebung ist, insbesondere wobei die axiale Eingriffsverschiebung eine Verschiebung ist, die entlang der Achse des ersten und des zweiten Verbinders (1, 11), insbesondere entlang der Achse des Verbindungssystems, gerichtet ist.

3. Verbindungssystem nach einem der vorhergehenden Ansprüche 1 und 2, wobei ein Umschalten aus dem verriegelten Zustand in den entriegelten Zustand durch den mindestens einen Verriegelungsvorsprung oder das mindestens eine Verriegelungsglied (14, 24) verhindert wird.

4. Verbindungssystem nach einem der vorhergehenden Ansprüche 1 - 2, wobei:
- die mindestens eine Aussparung des mindestens einen Verriegelungsglieds (14) des zweiten Verbinders (11) mindestens eine Öffnung ist und das mindestens eine Verriegelungsglied (24) des Bunds (20) mindestens einen Vorsprung umfasst, wobei der mindestens eine Vorsprung des Bunds (20) dazu ausgestaltet ist, in dem verriegelten Zustand in die Aussparung des zweiten Verbinders (11) einzutreten, um einen nicht entfernbaren axialen Eingriff zu definieren, insbesondere wobei sich der Vorsprung radial nach innen erstreckt, oder
- das mindestens eine Verriegelungsglied (14) des zweiten Verbinders (11) mindestens eine Vorsprung umfasst und die mindestens eine Aussparung des mindesten einen Verriegelungsglieds (24) des Bunds (20) eine Öffnung ist, wobei der mindestens eine Vorsprung des zweiten Verbinders (11) dazu ausgestaltet ist, in dem verriegelten Zustand in die Aussparung des Bunds (20) einzutreten, um einen nicht entfernbaren axialen Eingriff zu definieren, insbesondere wobei sich der Vorsprung radial nach außen erstreckt.

5. Verbindungssystem nach einem der vorhergehenden Ansprüche, wobei sich der eine oder die mehreren Finger (26) im Wesentlichen parallel zu der Achse des Bunds (20) erstrecken.

6. Verbindungssystem nach einem der vorhergehenden Ansprüche, wobei die Finger (26) des Bunds (20) die Vorsprünge des Bunds (20) tragen.

7. Verbindungssystem nach einem der vorhergehenden Ansprüche 1 - 6, wobei die Verriegelungsvorsprünge oder das Verriegelungsglied (24) des Bunds einen minimalen Innendurchmesser definieren, der kleiner als ein durch die Verriegelungsvorsprünge oder -glieder (14) des zweiten Verbinders (11) definierter maximaler Außendurchmesser ist, wobei die Finger (26) des Bunds (20) dazu ausgestaltet sind, sich während des Koppelns der Verriegelungsvorsprünge (14) des zweiten Verbinders mit den Verriegelungsvorsprüngen (24) des Bunds (20) radial nach außen zu biegen.

8. Verbindungssystem nach einem der vorhergehenden Ansprüche, wobei sich der Bund (20) in Bezug auf den zweiten Verbinder (11) frei dreht, wenn er in dem verriegelten Zustand angeordnet ist, wodurch gleichzeitig ein axiales Ausrücken zwischen dem Bund und dem zweiten Verbinder verhindert wird und somit ein axiales Ausrücken zwischen dem ersten und dem zweiten Verbinder verhindert wird.

9. Verbindungssystem nach den vorhergehenden Ansprüchen, wobei der zweite Verbinder (11) einen Hakenabschnitt (13) umfasst und der Bund einen jeweiligen Hakenabschnitt (23) umfasst, der durch eine Eingriffsdrehung entlang einer Eingriffsrichtung (ER) mit dem Hakenabschnitt (13) des zweiten Verbinders (11) in Eingriff bringbar ist, wobei der Hakenabschnitt (23) des Bunds (20) und der Hakenabschnitt (13) des zweiten Verbinders (11) in einer Eingriffskonfiguration ein axiales Entfernen des Bunds (20) von dem zweiten Verbinder (11) verhindern.

10. Verbindungssystem nach dem vorhergehenden Anspruch, wobei sowohl der Hakenabschnitt (23) des Bunds (20) als auch der Hakenabschnitt (13) des zweiten Verbinders jeweilige Gewinde umfassen, die dazu ausgestaltet sind, miteinander in Eingriff zu treten, um die Eingriffskonfiguration zu definieren, oder jeweilige hinterschnittene Verbindungen, die dazu ausgestaltet sind, als Bajonettkopplung miteinander in Eingriff zu treten, um die Eingriffskonfiguration zu definieren.

11. Verbindungssystem nach dem vorhergehenden Anspruch, wobei der Hakenabschnitt (13) des zweiten Verbinders (11) eine Nut zum Definieren der hinterschnittenen Verbindung, insbesondere einer Bajonettkopplung, umfasst,
und wobei die Nut einen ersten Kanal aufweist, der sich im Wesentlichen parallel zu der Achse des zweiten Verbinders erstreckt, und
- einen zweiten Kanal, der sich im Wesentlichen senkrecht zu dem ersten Kanal erstreckt, oder
- wobei der zweite Kanal bogenförmig ist und eine Konkavität aufweist, die einem Endabschnitt (12a) des zweiten Verbinders (11) zugewandt ist, wobei der Fluidkopplungsabschnitt (12) des zweiten Verbinders (11) einschließlich des Endabschnitts (12a) einen inneren Kanal (17) für den Fluidtransport definiert.

12. Verbindungssystem nach einem der vorhergehenden Ansprüche, wobei die axiale Ausrückverschiebung eine lineare Verschiebung in einer der axialen Eingriffsverschiebung entgegengesetzten Richtung ist.

13. Einwegsatz (50) für eine Blutbehandlungsvorrichtung, wobei der Einwegsatz Folgendes umfasst:
- einen Blutkreislauf (50a), aufweisend:
• eine Zuflussleitung (52), die dazu ausgestaltet ist, mit einem Patienten (300) und mit einem Einlass-Port (201a) einer Blutbehandlungseinheit (201) verbunden zu werden,
• eine Rückflussleitung (53), die dazu ausgestaltet ist, mit dem Patienten (300) und mit einem Auslass-Port (201b) der Blutbehandlungseinheit (201) verbunden zu werden,
- optional eine Infusionsleitung (54), die mit dem Blutkreislauf (52) verbunden und/oder dazu ausgestaltet ist, mit dem Patienten (300) verbunden zu werden,
- optional eine Überwachungsleitung (55), die mit dem Blutkreislauf (50a) oder mit der Infusionsleitung (54) verbunden ist,
- mindestens ein Verbindungssystem (100) nach einem der Ansprüche 1 bis 12, das mit der Zuflussleitung (52) und/oder der Rückflussleitung (53) und/oder der Infusionsleitung (54) und/oder der Überwachungsleitung (55) verbunden ist.

## Revendications

1. Système de connexion non amovible(100) pour un appareil médical, ledit système de connexion (100) comprenant:
- un premier connecteur(1) comprenant une partie de couplage de fluide(2), une partie de butée principale(5) agencée sur une paroi latérale externe du premier connecteur(1) et définissant une saillie s'étendant radialement vers l'extérieur à partir du premier connecteur (1), le premier connecteur(1) étant un connecteur mâle de type Luer;
- un second connecteur(11) comprenant une partie de couplage de fluide respective(12) pouvant être connectée, de manière étanche aux fluides, à la partie de couplage de fluide(2) du premier connecteur(1), de sorte qu'un fluide est apte à circuler intérieurement entre le premier et le second connecteur(1, 11), le second connecteur(11) comprenant en outre au moins un élément de verrouillage (14), le second connecteur(11) étant un connecteur femelle de type Luer;
- un collier(20) couplé au premier connecteur(1) et comprenant:
une partie de butée respective (25) configurée pour entrer en contact avec la partie de butée principale(5) du premier connecteur mâle(1) lorsqu'elle est disposée dans une configuration de butée, de sorte que le collier(20) est contraint axialement par rapport au premier connecteur(1) au moins selon une direction axiale;
au moins un élément de verrouillage respectif(24) engageable avec l'au moins un élément de verrouillage(14) du second connecteur(11) par un déplacement d'engagement axial le long d'une direction d'engagement(ED), dans une configuration couplée l'au moins un élément de verrouillage(24) du collier(20) et l'au moins un élément de verrouillage(14) du second connecteur(11) empêchant un déplacement axial de désengagement du collier(20) par rapport au second connecteur (11), ledit déplacement axial de désengagement étant un déplacement dans une direction opposée par rapport audit déplacement d'engagement axial, le collier(20) comprenant un corps principal de forme tubulaire s'étendant entre une première ouverture(21) configurée pour recevoir le second connecteur (11), et une seconde ouverture(22) recevant par insertion le premier connecteur(1), le collier(20) comprenant une pluralité de doigts(26) agencés au niveau de la première ouverture(21) répartis angulairement autour de l'axe du collier(20), lesdits doigts(26) portant l'au moins un élément de verrouillage(24) du collier(20);
et le système de connexion(100) étant configurable, par le déplacement d'engagement axial, depuis:
- un état déverrouillé, l'au moins un élément de verrouillage(24) du collier(20) et l'au moins un élément de verrouillage(14) du second connecteur(11) étant dans une configuration découplée; vers
- un état verrouillé, l'au moins un élément de verrouillage(24) du collier(20) et l'au moins un élément de verrouillage(14) du second connecteur(11) étant dans la configuration couplée, dans ledit état verrouillé, le collier(20) étant engagé de manière inamovible avec le second connecteur(11) par ledit déplacement d'engagement axial, et l'au moins un élément de verrouillage (14) du second connecteur(11) coopérant avec l'au moins un élément de verrouillage(24) empêchant le désengagement du premier connecteur(1) du second connecteur(11),
et dans lequel,
- l'au moins un élément de verrouillage(14) du second connecteur comprenant au moins un évidement et l'au moins un élément de verrouillage(24) du collier(20) comprenant au moins une saillie, l'au moins une saillie du collier(20) étant configurée dans l'état verrouillé pour engager l'évidement du second connecteur (11) pour définir un engagement axial non amovible, ou
- l'au moins un élément de verrouillage(14) du second connecteur(11) comprenant au moins une saillie, et l'au moins un élément de verrouillage (24) du collier(20) comprenant au moins un évidement, l'au moins une saillie du second connecteur(11) étant configurée dans l'état verrouillé pour engager l'évidement du collier(20) pour définir un engagement axial non amovible.

2. Système de connexion selon la revendication précédente, dans lequel le déplacement d'engagement axial étant un déplacement dirigé sensiblement parallèlement à l'axe des premier et second connecteurs(1, 11), en particulier ledit déplacement d'engagement axial étant un déplacement dirigé le long de l'axe des premier et second connecteurs(1, 11), en particulier le long de l'axe du système de connexion.

3. Système de connexion selon l'une quelconque des revendications 1 et 2, dans lequel un passage de l'état verrouillé à l'état déverrouillé étant empêché par l'au moins une saillie de verrouillage ou un élément de verrouillage(14, 24).

4. Système de connexion selon l'une quelconque des revendications 1 et 2, dans lequel:
- l'au moins un évidement de l'au moins un élément de verrouillage(14) du second connecteur(11) étant une ouverture, et l'au moins un élément de verrouillage(24) du collier(20) comprenant l'au moins une saillie, l'au moins une saillie du collier(20) étant configurée dans l'état verrouillé pour pénétrer dans l'évidement du second connecteur(11) pour définir un engagement axial non amovible, en particulier ladite saillie s'étendant radialement vers l'intérieur, ou
- l'au moins un élément de verrouillage(14) du second connecteur (11) comprenant l'au moins une saillie, et l'au moins un évidement de l'au moins un élément de verrouillage (24) du collier(20) étant une ouverture, l'au moins une saillie du second connecteur(11) étant configurée dans l'état verrouillé pour pénétrer dans l'évidement du collier(20) pour définir un engagement axial non amovible, en particulier ladite saillie s'étendant radialement vers l'extérieur.

5. Système de connexion selon l'une quelconque des revendications précédentes, dans lequel le ou les doigts(26) s'étendant sensiblement parallèlement à l'axe du collier(20).

6. Système de connexion selon l'une quelconque des revendications précédentes, dans lequel lesdits doigts(26) du collier(20) portant les saillies du collier (20) .

7. Système de connexion selon l'une quelconque des revendications 1 à 6, dans lequel les saillies ou l'élément de verrouillage(24) du collier définissant un diamètre intérieur minimal inférieur à un diamètre extérieur maximal défini par les saillies ou l'élément de verrouillage(14) du second connecteur(11), dan lequel lesdits doigts(26) du collier(20) étant configurés pour fléchir radialement vers l'extérieur pendant le couplage des saillies de verrouillage(14) du second connecteur avec les saillies de verrouillage(24) du collier(20).

8. Système de connexion selon l'une quelconque des revendications précédentes, dans lequel le collier (20) tournant librement par rapport au second connecteur (11) lorsqu'il est agencé dans l'état verrouillé, empêchant en même temps un désengagement axial entre le collier et le second connecteur, et empêchant ainsi un désengagement axial entre le premier et le second connecteur.

9. Système de connexion selon les revendications précédentes, dans lequel le second connecteur(11) comprenant une partie d'accrochage (13), et le collier comprenant une partie d'accrochage respective(23) pouvant être engagée avec la partie d'accrochage(13) du second connecteur(11) par l'intermédiaire d'une rotation d'engagement le long d'une direction d'engagement (ER), dans une configuration engagée, la partie d'accrochage(23) du collier(20) et la partie d'accrochage(13) du second connecteur(11) empêchant le retrait axial du collier (20) du second connecteur(11).

10. Système de connexion selon la revendication précédente, dans lequel la partie d'accrochage(23) du collier(20) et la partie d'accrochage(13) du second connecteur comprenant toutes deux des filetages respectifs configurés pour venir en prise l'un avec l'autre afin de définir la configuration engagée; ou des joints en contre-dépouille respectifs configurés pour venir en prise l'un avec l'autre en tant que couplage à baïonnette afin de définir la configuration engagée.

11. Système de connexion selon la revendication précédente, dans lequel la partie d'accrochage(13) du second connecteur(11) comprenant une rainure pour définir le joint en contre-dépouille, en particulier un couplage à baïonnette,
et dans lequel ladite rainure comprenant un premier tronçon s'étendant sensiblement parallèlement à l'axe du second connecteur, et
- un second tronçon s'étendant sensiblement perpendiculairement au premier tronçon, ou
- ledit second tronçon étant de forme arquée avec une concavité tournée vers une partie terminale(12a) du second connecteur(11), la partie de couplage de fluide(12) du second connecteur (11) comprenant ladite partie terminale (12a) définissant un canal interne(17) pour le transport de fluide.

12. Système de connexion selon l'une quelconque des revendications précédentes, dans lequel ledit déplacement axial de désengagement étant un déplacement linéaire dans une direction opposée par rapport audit déplacement d'engagement axial.

13. Ensemble jetable(50) pour un appareil de traitement du sang, ledit ensemble jetable comprenant:
- un circuit sanguin(50a) comprenant:
• une ligne d'accès(52) configurée pour être raccordée à un patient(300) et à un orifice d'entrée(201a) d'une unité de traitement sanguin(201),
• une ligne de retour(53) configurée pour être raccordée au patient(300) et à un orifice de sortie(201b) de l'unité de traitement sanguin(201),
- éventuellement une ligne de perfusion(54) connectée au circuit sanguin(52) et/ou configurée pour être connectée au patient(300);
- éventuellement une ligne de surveillance(55) connectée au circuit sanguin(50a) ou à la ligne de perfusion(54) ;
- au moins un système de connexion(100) selon l'une quelconque des revendications 1 à 12, connecté à au moins l'une des lignes parmi la ligne d'accès(52), la ligne de retour(53), la ligne de perfusion(54) et la ligne de surveillance(55).
